# EUROPÄISCHE PATENTSCHRIFT

(11) **EP 3 005 937 B1**
(45) Veröffentlichungstag und Bekanntmachung des Hinweises auf die Patenterteilung: **28.02.2018**
(21) Anmeldenummer: 15186908.8
(22) Anmeldetag: 25.09.2015
(51) Int. Cl.: G01B 9/02, A61B 3/10, A61B 3/12, A61B 3/13, A61B 5/00, A61B 90/20, G02B 21/36, G06T 5/00, G06T 5/50, A61B 34/20, A61B 90/00

(54) **VERFAHREN ZUR KORREKTUR EINES OCT-BILDES UND MIKROSKOP**
METHOD FOR CORRECTING AN OCT IMAGE AND MICROSCOPE
PROCÉDÉ DE CORRECTION D'UNE IMAGE OCT ET MICROSCOPE

(30) Priorität: 25.09.2014 DE 102014113901
(43) Veröffentlichungstag der Anmeldung: 13.04.2016
(60) Teilanmeldung: 16185203.3
(73) Patentinhaber: Carl Zeiss Meditec AG, 07745 Jena (DE)
(72) Erfinder: ESLAMI, Abouzar, 81479 München (DE); FILIPPATOS, Konstantinos, 80802 München (DE); MAIER-MATIC, Corinna, 82061 Neuried (DE); KOCHWAGNER, Christine, 83543 Rott am Inn (DE); SCHUHRKE, Thomas, 81539 München (DE); MECKES, Günter, 81477 München (DE); DUCA, Stefan, 85586 Poing (DE); SCHIELE, Carolin, 73441 Aufhausen (DE); HARTWIG, Falk, 81475 München (DE)
(74) Vertreter: Patentanwälte Geyer, Fehners & Partner mbB

(56) Entgegenhaltungen:
- WO-A1-2013/141155
- WO-A2-2012/100030
- US-A1- 2010 189 334
- US-A1- 2012 063 660
- US-A1- 2012 200 824
- US-A1- 2013 197 357
- US-A1- 2014 100 439
- US-A1- 2014 100 440

## Beschreibung

Die Erfindung bezieht sich auf ein Verfahren zum Verbessern eines OCT-Bildes eines Objekts, insbesondere der Netzhaut eines Auges, wobei eine Zeitreihe von OCT-Bildern bereitgestellt wird, die vom Objekt mittels optischer Kohärenztomographie durch einen Abbildungsstrahlengang erzeugt wurden, und wobei das Verfahren zum Verbessern eines OCT-Bildes, um Abschattungen durch einen im Abbildungsstrahlengang bewegten Gegenstand, insbesondere ein Operationsinstrument, zu unterdrücken, OCT-Bilder der Zeitreihe kombiniert.

Die Erfindung bezieht sich ebenfalls auf ein Mikroskop zur Abbildung eines Objekts, insbesondere der Netzhaut eines Auges, wobei das Mikroskop als Kombinationsmikroskop ausgebildet ist, welches einen Abbildungsstrahlengang, der das Objekt mittels optischer Kohärenztomographie abbildet, und eine Bildbearbeitungseinrichtung zur Unterdrückung von Abschattungen durch einen in einem OCT-Abbildungsstrahlengang bewegten Gegenstand, insbesondere ein Operationsinstrument, in einer Zeitreihe von OCT-Bildern aufweist.

Aus der WO 2012/100030 A2 ist ein Operationsmikroskopiesystem bekannt, in das ein OCT-System integriert ist. Damit kann ein Objektfeld optisch abgebildet werden, wobei das optisch erzeugte Bild über Okulare einer Abbildungsoptik betrachtet werden kann. Das OCT-System führt scannend am Objektfeld Messungen mittels optischer Kohärenztomographie durch. Dieses Operationsmikroskopiesystem wird insbesondere dafür eingesetzt, während eines chirurgischen Eingriffes am Auge das Auge sowohl optisch als auch durch OCT-Bilder abzubilden. Bei dieser Anwendung kann der Fall eintreten, dass ein Operationsinstrument Teile des Bildes abschattet. Die genannte Druckschrift sieht deshalb ein Verfahren sowie ein Operationsmikroskop der eingangs genannten Art vor, um Abschattungen im OCT-Bild zu unterdrücken. Dabei ist es vorgesehen, eine Zeitreihe von OCT-Bildern zu erzeugen und diese OCT-Bilder zu mitteln. Bewegungen des Instruments vor dem Objekt und die damit durch das Instrument verursachten Abschattungen sind damit unterdrückt.

Ein ähnliches Operationsmikroskopiesystem, das ebenfalls eine Kombination aus optischer Mikroskopie und OCT-Bildgebung ermöglicht ist aus der US 2014/0024949 A1 bekannt.

Aus der US 2013/0208129 A1 ist ein Verfahren zur digitalen Bildverbesserung bekannt. Die Veröffentlichung schlägt vor, zur Rauschreduzierung die Signale einzelner Pixel einer Schwellwertüberprüfung zu unterwerfen. Für Pixel, deren Rauschen oberhalb eines Referenzwertes liegt, werden Pixeldaten aus älteren Bildern gesucht, die unterhalb der Rauschgrenze liegen, und als Ersatzinformation eingefügt.
US 2014/100439 A1 beschreibt ein OCT-Verfahren zur Abbildung einer Wand eines Blutgefäßes mit einem in dem Blutgefäß angeordneten OCT-Messkopf. Es ist dabei das Ziel, ein komplettes OCT-Bild der Wand bereitzustellen, obgleich ein komplettes OCT-Bild aufgrund eines zur Führung des Messkopfs vorhandenen Drahtes nicht erzeugt werden kann. Daher werden mindestens zwei OCT-Bilder für jeweils unterschiedliche Positionen des Führungsdrahtes aufgenommen, und der in einem OCT-Bild durch den Führungsdraht abgeschattete Bereich wird durch die entsprechende Bildinformation aus einem der anderen OCT-Bilder ersetzt. US 2012/200824 A1 beschreibt ein OCT-Mikroskop, bei dem OCT-Bilder aus unterschiedlichen Winkeln aufgenommen werden und in einem Prozessor zu einem Gesamtbild zusammengefügt werden.
Der Erfindung liegt die Aufgabe zugrunde, ein Verfahren und ein Mikroskop der eingangs genannten Art dahingehend weiterzubilden, dass Abschattungen durch einen im Abbildungsstrahlengang bewegten Gegenstand, insbesondere ein Kombinationsmikroskop bei der Abbildung der Netzhaut eines Auges, besser unterdrückt sind.
Die Erfindung ist in den unabhängigen Ansprüchen 1 und 8 definiert.
Die Erfindung sieht vor, ein OCT-Mikroskopieverfahren bzw. OCT-Mikroskopbild hinsichtlich Bildfehlern zu korrigieren, die dadurch entstehen, dass Bereiche des Bildes durch einen Gegenstand, der im Abbildungsstrahlengang bewegt wird, (beispielsweise ein chirurgisches Instrument) abgeschattet werden. Die Erfindung gleicht die Wirkung eines Schattens im OCT-Bild dadurch aus, dass in einer Zeitreihe von OCT-Bildern ein anderes OCT-Bild gesucht wird, in dem der von der Abschattung betroffene Bereich nicht vom Gegenstand abgeschattet wird. Anders als im Konzept der US 2013/0208129 A1 wird also nicht das Rauschen in Bildbestandteilen ausgewertet, sondern es wird überprüft, ob ein Teil des OCT-Bildes durch den Gegenstand abgeschattet ist. Diese Abschattung äußert sich nicht in einem Bildrauschen, wäre also mit dem Ansatz gemäß US 2013/0208129 A1 gar nicht zu erkennen. Weiter wird für den Ersatz des abgeschatteten Bereiches nicht nach Bildern gesucht, die bestimmte Rauscheigenschaften erfüllen, sondern nach Bildern, in denen der Gegenstand den betroffenen Bereich nicht abschattet.

Die Abschattung kann in Ausführungsformen mit einer Schwellwertüberprüfung erkannt werden. Der entsprechende Referenzwert hat jedoch nichts mit dem Rauschen zu tun. Vielmehr ist in diesen Ausführungsformen der Referenzwert, der bei der Schwellwertüberprüfung zugrunde gelegt wird, genau so gewählt, dass damit eine Abschattung durch den Gegenstand, d. h. das Operationsinstrument, erkannt wird. Es ist deshalb für einige Ausführungsformen bevorzugt, dass bei der automatischen Suche der abgeschatteten Bereiche ein Referenzwert verwendet wird/ist, der genau so gewählt wird/ist, dass damit ein Schatten, den der Gegenstand (z. B. ein Operationsinstrument) wirft, erkannt wird.

Die Bildinformation des anderen OCT-Bildes für diesen Bereich wird verwendet, um das zu korrigierende Bild hinsichtlich der Abschattung zu verbessern. Diese Art der Bildkorrektur ist weitaus besser, als die aus dem Stand der Technik bekannte Mittelung, da nur noch Bildinformationen kombiniert werden, welche von der Abschattung durch den Gegenstand nicht beeinträchtigt wurden. Ein Benutzer erhält damit in allen Bildbereichen, auch in solchen, die sich im Schatten des Gegenstandes befinden, die maximale Bildqualität.

Der Begriff "Abschattung" ist in dieser Beschreibung auf die Schattenwirkung des Gegenstandes bei der OCT-Abbildung bezogen. Bei einem Kombinationsmikroskop, das optische Mikroskopie und OCT-Mikroskopie kombiniert, ist in der Regel die Abschattung, die der Gegenstand für das OCT-Bild erzeugt, nicht identisch mit dem Schatten im optischen Bild. Dies gilt ganz besonders für die Abbildung der Netzhaut eines Auges, da dort in der optischen Abbildung üblicherweise eine streifende Beleuchtung verwendet wird, die unter einem völlig anderen Winkel einfällt, als die Strahlung bei der OCT-Abbildung.

Die Abschattung gibt es natürlich nur dann, wenn der Gegenstand für die Strahlung, welche zur Erzeugung des OCT-Bildes verwendet wird, nicht transparent oder nicht ausreichend transparent ist. Unter dem Begriff "Gegenstand" wird deshalb in dieser Beschreibung ein Gegenstand verstanden, der die für die Erzeugung des OCT-Bildes verwendete Strahlung absorbiert.

Das erfindungsgemäße Verfahren ermittelt die Abschattung im OCT-Bild durch den Gegenstand. Dies erfordert jedoch auch bei Bildern von menschlichem Gewebe nicht zwingend, dass diese Ermittlung während eines chirurgischen Eingriffs durchgeführt wird. Das Verfahren kann vielmehr auch an bereits erzeugten OCT-Bildern zeitlich nach einem chirurgischen Eingriff oder an OCT-Bildern von totem Geweben oder technischem Material ausgeführt werden. Das Verfahren ist deshalb nicht chirurgisch und erfordert insbesondere nicht die Mitwirkung eines Arztes oder Therapeuten. Vielmehr ist es möglich, das Verfahren an bereits anderweitig erzeugten und bereitgestellten OCT-Bildern auszuführen.

Das Verfahren bzw. die Bildbearbeitungseinrichtung sucht in der Zeitreihe nach mindestens einem anderen Bild, in dem der Bereich des Objektes, der im zu korrigierenden Bild abgeschattet ist, nicht vom Gegenstand abgeschattet wird. Für den Fall eines konstant während der Zeitreihe ruhenden Gegenstandes, gibt es ein solches anderes Bild nicht. In diesem Fall wird das Verfahren mit einer Fehlermeldung beendet bzw. die Bildbearbeitungsvorrichtung markiert den abgeschatteten Bereich im nicht korrigierten Bild, beispielsweise durch eine Schwärzung etc.

Eine Markierung ist auch möglich, falls das Verfahren die Bildverbesserung ausführt bzw. die Bildbearbeitungsvorrichtung ein korrigiertes, also verbessertes OCT-Bild erzeugte. Auf diese Weise erfährt ein Benutzer, welche Bereiche des korrigierten OCT-Bildes möglicherweise unaktuelle Bildinformation enthalten. Es ist deshalb in einer Ausgestaltung des Verfahrens bevorzugt, dass im korrigierten OCT-Bild der Bereich des Objektes, für den die Bildinformation eingefügt wurde, markiert wird. Analog ist es für das Kombinationsmikroskop bevorzugt, dass die Bildbearbeitungseinrichtung im korrigierten OCT-Bild den Bereich des Objektes, in dem die Bildinformation eingefügt ist, markiert.

Diese Markierung kann in einer weiteren Ausführungsform der Erfindung dahingehend weitergebildet werden, dass sie eine Anzeige über den zeitlichen Abstand zwischen dem zu korrigierenden OCT-Bild, in dem die Einfügung erfolgte, und dem anderen OCT-Bild, aus dem die eingefügte Information stammte, bereitstellt. Diese Anzeige kann in einer bevorzugen Ausführungsform eine Markierung, insbesondere eine Graustufen- oder Farbdarstellung des Bereiches, in den die Information eingefügt ist, umfassen. So ist es beispielsweise möglich, mit zunehmendem zeitlichen Abstand bei einer Schwarzweißdarstellung den eingefügten Bereich mit zunehmend geringerem Kontrast zu gestalten oder mit zunehmend geringerer Helligkeit/Schwärze. Ein Betrachter nimmt damit intuitiv wahr, dass die Bildinformation in diesem Abschnitt des Bildes weniger verlässlich, weil weniger aktuell ist.

Bei der Suche nach dem anderen OCT-Bild, in dem der Bereich des Objektes, der im zu korrigierenden OCT-Bild abgeschattet ist, nicht abgeschattet ist, wählt eine bevorzugte Ausführungsform, falls es mehrere solcher Bilder gibt, dasjenige andere OCT-Bild aus, welches den geringsten zeitlichen Abstand zum zu korrigierenden OCT-Bild hat. In der Regel ist das andere Bild zeitlich älter als das zu korrigierende Bild, und es wird im Fall, dass mehrere andere Bilder in Frage kommen, bevorzugt das jüngste dieser anderen Bilder ausgewählt.

In einer anderen Ausführungsform wird zusätzlich ein Korrekturinformationsbild erzeugt, das den Bereich des Objektes, in dem die Bildinformation als Korrektur eingefügt wurde, anzeigt. Es kann sich dabei um ein stark vereinfachtes OCT-Bild des Objektes handeln, in dem der Bereich, in den die Information eingefügt ist, markiert ist.

Die Erfindung kommt bei der Kombination von optischer Mikroskopie und OCT-Bildgebung, wie sie im eingangs genannten Operationsmikroskop gemäß US 2014/0024949 A1 beschrieben ist, zum Einsatz. Dabei wird anhand des optischen Bildes die Lage des Gegenstandes und damit die Lage des Bereiches des Objektes, der im OCT-Bild abgeschattet ist, ermittelt. Es ist deshalb vorgesehen, das Objekt zusätzlich optisch abzubilden und so ein optisches Bild des Objektes zu erzeugen, wobei die Lage des Gegenstandes im OCT-Bild anhand des optischen Bildes festgestellt wird und daraus der Bereich des Objektes ermittelt wird, den der Gegenstand im OCT-Bild abschattet. Das Kombinationsmikroskop weist daher eine Kamera auf, die ein optisches Bild des Objektes erzeugt und Optikbilddaten bereitstellt, wobei die Bildbearbeitungseinrichtung die Optikbilddaten ausliest, die Lage des Gegenstandes im optischen Bild feststellt und daraus den Bereich des Objektes ableitet, der durch den Gegenstand im zu korrigierenden Bild abgeschattet ist.

Um beim fortlaufenden Betrieb des Kombinationsmikroskops bzw. im Verfahren zu verbesserndes OCT-Bildes bei fortlaufender Bildgebung möglichst rechensparsam und schnell die Bildinformation für die von der Abschattung im OCT-Bild betroffenen Bereiche des Objektes zur Verfügung zu haben, ist es in einer weiteren Ausführungsform bevorzugt, parallel zur Zeitreihe von OCT-Bildern eine Abfolge von optischen Bildern zu erzeugen und daraus eine Folge von Angaben über den Bereich des Objektes, das durch den Gegenstand im OCT-Bild abgeschattet wird, zu ermitteln, wobei jede Angabe der Folge einem OCT-Bild der Zeitreihe zugeordnet ist. Für den Fall der nachträglichen Ausführung des Verfahrens zum Verbessern eines OCT-Bildes ist es in einer Ausführungsform vorgesehen, dass eine Abfolge von optischen Bildern bereitgestellt wird, die parallel zur Zeitreihe von OCT-Bildern erzeugt wurde, wobei im Verfahren aus der Abfolge von optischen Bildern eine Folge von Angaben über den Bereich des Objektes, der durch den Gegenstand im OCT-Bild abgeschattet wird, ermittelt wird, wobei jede Angabe der Folge einem OCT-Bild der Zeitreihe zugeordnet wird.

Auf diese Weise kann sehr schnell das jüngste andere OCT-Bild gefunden werden. Die Suche beschränkt sich zumeist darauf, dass jüngste OCT-Bild zu finden, in dem der Bereich des Objektes, der im zu korrigierenden OCT-Bild von der Abschattung betroffen ist, nicht abgeschattet ist. Aufgrund der Angabe über den Bereich des Objektes, der durch den Gegenstand im OCT-Bild abgeschattet ist, die für jedes OCT-Bild der Zeitreihe vorliegt, ist die Suche besonders schnell und rechensparsam.

Die Erfindung ist natürlich nicht darauf beschränkt, das OCT-Bild hinsichtlich nur eines einzigen Gegenstandes zu verbessern. Es ist gleichermaßen möglich die Verbesserung auch für mehrere Gegenstände, beispielsweise zwei bei einer chirurgischen Operation zusammenwirkende Instrumente etc. auszuführen.

Für den Fall, dass das Verfahren während der Bildgebung, also online, eingesetzt werden soll, ist in einer weiteren Ausführungsform vorgesehen: ein Verfahren zur Abbildung eines Objekts, insbesondere der Netzhaut eines Auges, wobei mittels optischer Kohärenztomographie durch einen Abbildungsstrahlengang ein OCT-Bild des Objekts erzeugt wird und, um Abschattungen durch einen im Abbildungsstrahlengang bewegten Gegenstand, insbesondere ein Operationsinstrument, zu unterdrücken, eine Zeitreihe von OCT-Bildern erzeugt und OCT-Bilder der Zeitreihe kombiniert werden, wobei für ein zu korrigierendes OCT-Bild ein in diesem OCT-Bild liegender Bereich des Objektes ermittelt wird, der durch den Gegenstand abgeschattet wird, in der Zeitreihe ein anderes OCT-Bild gesucht wird, in dem der Bereich des Objektes nicht durch den Gegenstand abgeschattet ist, aus diesem anderes OCT-Bild für der Bereich des Objektes Bildinformation ausgelesen wird und ein korrigiertes OCT-Bild erzeugt wird, indem in das zu korrigierende OCT-Bild die ausgelesene Bildinformation eingefügt wird, wobei die ausgelesene Bildinformation im zu korrigierenden OCT-Bild den Bereich des Objektes, der durch den Gegenstand abgeschattet wird, ersetzt.

Es versteht sich, dass die vorstehend genannten und die nachstehend noch zu erläuternden Merkmale nicht nur in den angegebenen Kombinationen, sondern auch in anderen Kombinationen oder in Alleinstellung gemäß dem Schutzumfang der unabhängigen Ansprüche einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Darstellung eines Mikroskopiesystems,
- Fig. 2: eine Schemadarstellung dreier OCT-Bilder, die mit dem Mikroskop 1 der Fig. 1 erzeugt werden und hinsichtlich der Abschattung durch einen im Abbildungsstrahlengang bewegten Gegenstand verbessert werden,
- Fig. 3: ein Korrekturinformationsbild, das einem Benutzer eine Information über die betroffenen Bereiche der Bildverbesserung gibt,
- Fig. 4: ein Blockschaltbild bezüglich der Bildverbesserung und
- Fig. 5: ein Ablaufdiagramm des Verfahrens zur Bildverbesserung.

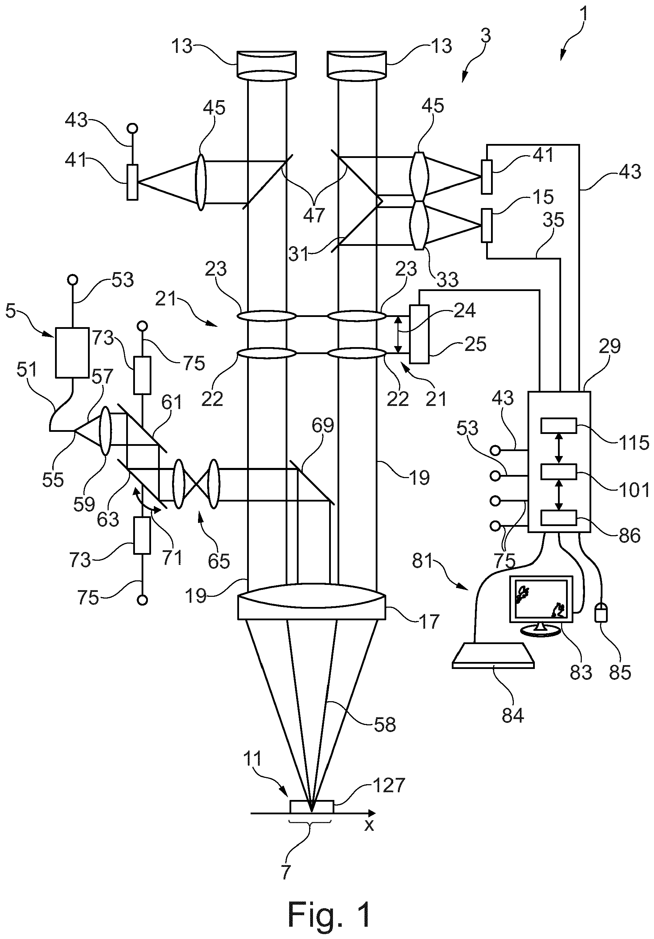

Ein in Fig. 1 schematisch dargestelltes Mikroskopiesystem 1 umfasst eine Abbildungsoptik 3 und ein OCT-System 5. Das Mikroskopiesystem 1 ist ein Kombinationsmikroskop, z.B. ein Operationsmikroskop.

Die Abbildungsoptik 3 bildet ein Objekt 7 in einem Objektfeldes 11 zum einen auf ein Okular 13 und zum anderen auf eine Kamera 15 ab. Die Abbildungsoptik 3 umfasst eine Objektivlinse 17, welche aus ein oder mehreren Linsenelementen bestehen kann und, in dem hier dargestellten Beispiel, das Objektfeld 11 nach Unendlich abbildet. In dem Strahlengang hinter der Objektivlinse 17 werden zwei Strahlenbündel 19 durch jeweils eine Zoomlinsenanordnung 21 geführt, welche einen Abbildungsmaßstab der Abbildungsoptik 3 ändern können. Die beiden Zoomlinsenanordnungen weisen jeweils mindestens zwei Linsengruppen 22, 23 auf, welche relativ zueinander in Strahlrichtung der Bündel 19 verschieblich sind, wie dies in Fig. 1 durch einen Pfeil 24 angedeutet ist. Die Verschiebung der beiden Linsengruppen 22, 23 relativ zueinander wird durch einen Aktuator 25 bewirkt, welcher zur Einstellung des Abbildungsmaßstabs der Abbildungsoptik 3 über eine Steuerleitung 27 von einer Steuereinrichtung 29 angesteuert wird.

Nach Durchlaufen der Zoomlinsenanordnungen 21 treten die Strahlbündel 19 in das Okular 13 ein. Aus dem in Fig. 1 rechts gezeigten Strahlenbündel 19 koppelt ein teildurchlässiger Spiegel 31 ein Teil der Strahlung aus. Er wird über eine Kameraadapteroptik 33 auf die Kamera 15 geführt, so dass diese ein optisches Bild des Objekts 7 detektiert. Von der Kamera 15 erzeugte Optikbilddaten werden über eine Datenleitung 35 an die Steuereinrichtung 29 übertragen.

Die Abbildungsoptik 3 umfasst in der gezeigten Ausführungsform zwei (grundsätzlich optionale) elektronische Anzeigeeinrichtungen 41, welche von der Steuereinrichtung 29 über Datenleitungen 43 mit Anzeigebilddaten versorgt werden. Von den Anzeigeeinrichtungen 41 erzeugte Anzeigebilder werden jeweils über eine Projektionsoptik 45 und in den Strahlenbündeln 19 angeordneten teildurchlässigen Spiegeln 47 in die Strahlengänge hin zu den Okularen 13 projiziert, so dass ein Benutzer, der in die Okulare 13 Einblick nimmt, die durch die Anzeigeeinrichtungen 41 dargestellten Bilder in Überlagerung mit dem Bild des Objekts 7 wahrnehmen kann.

Das OCT-System 5 umfasst eine zur Erzeugung von OCT-Bildinformation geeignete kurzkohärente Lichtquelle (Weißlichtquelle) und ein Interferometer, welche in Fig. 1 nur schematisch als Block dargestellt sind. Über eine Lichtleitfaser 51 gibt das OCT-System 5 OCT-Beleuchtungsstrahlung 57 ab, die auf ein zu vermessendes Objekt treffen kann. Vom Objekt in Gegenrichtung zurückkommende OCT-Messstrahlung wird an der Lichtleitfaser 51 eingekoppelt.

Die aus einem Ende 55 der Lichtleitfaser 51 austretende OCT-Beleuchtungsstrahlung 57 wird dabei durch eine Kollimatoroptik 59 zu einem OCT-Beleuchtungsstrahl 58 kollimiert, an zwei Ablenkspiegeln 61 und 63 abgelenkt, durchläuft eine Projektionsoptik 65, trifft auf einen Spiegel 69 und wird von diesem durch die Objektivlinse 17 auf das Objektfeld 11 gerichtet. Die von dem in dem Objektfeld 11 angeordneten Objekt 7 zurückgeworfene OCT-Messstrahlung durchläuft den umgekehrten Weg durch die Objektivlinse 17, die Projektionsoptik 65 und die Kollimatoroptik 59 und wird in die Lichtleitfaser 51 eingekoppelt.

Das OCT-System 5 wertet mittels des Interferometers die OCT-Messstrahlung interferometrisch aus und erzeugt ein OCT-Bild, je nach noch zu erläuterndem Scanprinzip als sog. A-Scan, B-Scan oder C-Scan. Das OCT-System 5 wird von der Steuereinrichtung 29 über eine Steuer- und Datenleitung 53 kontrolliert, und sie empfängt über diese Leitung auch die OCT-Bilddaten von dem OCT-System 5.

Die Spiegel 61 und 63 sind schwenkbar, um den OCT-Beleuchtungsstrahl 58 auf verschiedene Orte des Objektfeldes 11 abzulenken und die OCT-Messstrahlung von diesen Orten aufzusammeln, also mittels der Spiegel 61, 63 das Objektfeld 11 mindestens teilweise abzuscannen. Wie durch einen Pfeil 71 angedeutet, wirkt der Spiegel 63 als Scanspiegel in x-Richtung (Horizontalrichtung in Fig. 1), der Spiegel 61 als Scanspiegel in y-Richtung (senkrecht zur Zeichenebene in Fig. 1). Die Spiegel 61 und 63 werden von Aktuatoren 73 eingestellt, welche von der Steuereinrichtung 29 über Steuerleitungen 75 gesteuert werden. Die Steuereinrichtung 29 kann somit durch entsprechende Ansteuerung der Aktuatoren 73 den OCT-Messstrahl entlang eines einstellbaren Scanpfades über das Objektfeld führen.

Die Steuereinrichtung 29 umfasst in einer Ausführungsform eine graphische Benutzerschnittstelle 81, welche einen Bildschirm 83 als Darstellungsmedium, eine Tastatur 84 und eine Maus 85 als Eingabemedien und ein Modul 86 zur Steuerung des Mikroskopiesystem 1 aufweist, welches z.B. in der Steuereinrichtung 29 als Software-Modul läuft.

Die Funktionsweise des OCT-Systems 5 ist für die nachfolgend erläuterte Bildverbesserung nicht weiter von Bedeutung. Insbesondere kann das OCT-System 5 als SS-OCT, SD-OCT, FT-OCT oder TD-OCT ausgebildet sein, wie es im Stand der Technik für OCT-Systeme bekannt ist. Das OCT-System 5 erzeugt ein tiefenaufgelöstes Bild 127 des Objektes 7 im Objektfeld. Aufgrund des interferometrischen Prinzips des OCT-Systems 5 wird an jedem Auftreffpunkt des OCT-Beleuchtungsstrahls 58, von dem auch die OCT-Messstrahlung aufgesammelt wird, ein tiefenaufgelöstes Bild 127 erzeugt, das jedoch ohne Verschiebung des Auftreffpunktes nur eindimensional ist, also Tiefeninformation entlang der optischen Achse liefert, welche durch die Einstellung der Ablenkspiegel 61 und 63 vorgegeben ist. Ein solches eindimensionales Bild wird als A-Scan bezeichnet. Lenkt man den Auftreffpunkt des OCT-Beleuchtungsstrahls 58 längs einer Linie ab, ergibt sich ein linienförmiges Bild 127, welches als B-Scan bezeichnet wird. Verschiebt man diese Linie quer zur Linienrichtung, erhält man ein Volumenbild oder einen sogenannten C-Scan. Das Bild 127 ist dann de facto eine 3D-Auflösung des Objektes 7 im Bereich, der durch die Verstellung der Ablenkspiegel 61 und 63 abgetastet wird.

In der in Fig. 1 gezeigten Ausführungsform weist das Operationsmikroskop 1 neben dem Modul 86, welches die Grundfunktionen des Mikroskopiesystems 1 steuert, noch ein optisches Bildmodul 101 sowie ein OCT-Bildmodul 115 auf. Das optische Bildmodul 101 empfängt Bilddaten von der Kamera 15 und stellt ein optisches Bild bereit. Das OCT-Bildmodul 115 empfängt OCT-Bilddaten vom OCT-System 5 und stellt ein OCT-Bild bereit. Dabei erzeugen OCT-Bildmodul 115 und optisches Bildmodul 101 fortlaufend die entsprechenden Bilder, so dass eine Zeitreihe von OCT-Bildern und eine Abfolge von optischen Bildern zur Verfügung steht, die in einer bevorzugten Ausführungsform jeweils gleichzeitig gewonnen wurden, so dass jedes OCT-Bild der Zeitreihe jeweils einem optischen Bild der Folge entspricht. Die Bildmodule 101 und 115 speichern diese Zeitreihen ab, und ein Benutzer kann sie über die graphische Benutzerschnittstelle 81 abrufen.

Beim Einsatz des Mikroskopiesystems 1 als Operationsmikroskop wird ein chirurgisches Instrument zur Manipulation des Objektes 7 eingesetzt. Dieses chirurgische Instrument ist ein Beispiel für einen Gegenstand, der im Strahlengang zwischen Objekt 7 und Objektivlinse 17 bewegt wird, im Falle der Augenchirurgie sogar innerhalb des Objektes 7, beispielsweise im Bereich der Netzhaut.

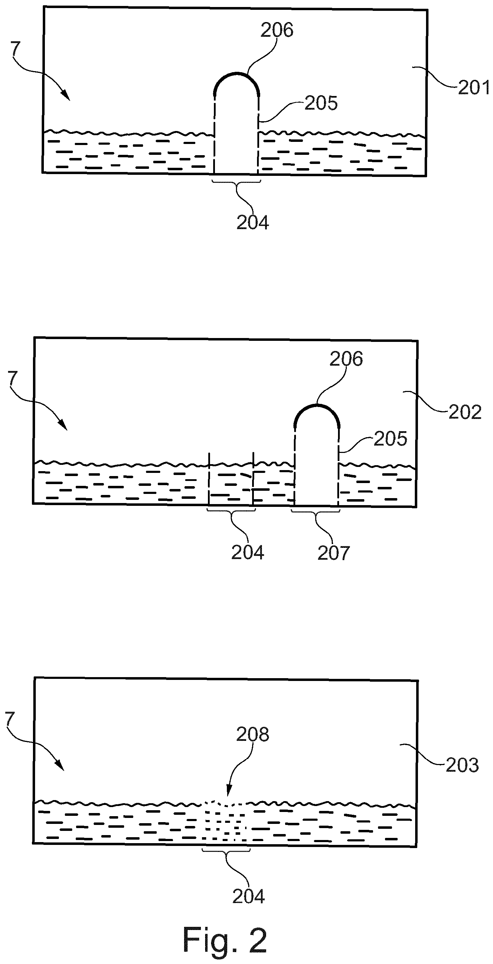

Fig. 2 zeigt drei OCT-Bilder übereinander, in denen ein Instrument 206 eine Rolle spielt. Im oberen Bild der Fig. 2 ist ein aktuelles OCT-Bild 201 dargestellt, das eine Schnittdarstellung des Objektes 7 zeigt. Zu sehen ist eine Grenzfläche (symbolisiert durch eine Wellenlinie) sowie tieferliegende Schichten (symbolisiert durch eine Strichlierung). Im OCT-Bild 201 befindet sich ein Instrument 206. Dieses ist für die OCT-Beleuchtungsstrahlung 58 nicht transparent. Folglich wirft das Instrument 206 einen Schatten 205, der einen Bereich 204 des Objektes 7 abschattet. In diesem abgeschatteten Bereich 204 ist keine Information im OCT-Bild vorhanden. Vielmehr sieht man bloß die der Objektivlinse 7 zugewandte Grenzfläche des Instrumentes 206, nicht jedoch darunterliegende Bereiche. Das oberste Bild der Fig. 2 ist deshalb ein zu korrigierendes OCT-Bild 201, das hinsichtlich der Abschattungswirkung des Instrumentes 206 korrigiert werden soll.

Die Steuereinrichtung weist (in Fig. 1 nicht weiter dargestellte) Module, z.B. Softwaremodule, auf, welche in der vom OCT-Bildmodul 115 bereitgestellten Zeitreihe ein anderes OCT-Bild 202 suchen. Bei einem online-Betrieb handelt es sich beim anderen OCT-Bild 202 in der Regel um ein älteres OCT-Bild, also ein OCT-Bild, das in der Zeitreihe, welche vom OCT-Bildmodul 115 bereitgestellt wird, vor dem zu korrigierenden OCT-Bild 201 aufgenommen wurde. Das andere OCT-Bild, welches von der Steuereinrichtung 29 gesucht wird, ist dadurch gekennzeichnet, dass es das Objekt 7 in dem Bereich 204, der im zu korrigierenden OCT-Bild 201 abgeschattet ist, nicht abgeschattet zeigt. Dies ist im mittleren Bild der Fig. 2 exemplarisch als Situation dargestellt, in der das Instrument 206 an einer anderen Stelle im anderen OCT-Bild 202 liegt, als im zu korrigierenden OCT-Bild 201. Der im anderen OCT-Bild 202 abgeschattete Bereich 207 ist damit nicht identisch (Mindestvoraussetzung) oder überlappt überhaupt nicht (Optimalvoraussetzung) mit dem Bereich 204 des Objektes 7, der im zu korrigierenden OCT-Bild 201 abgeschattet ist.

Die Steuereinrichtung 29 liest für den abgeschatteten Bereich 204 Bildinformation 208 aus dem anderen OCT-Bild 202 aus und setzt sie an der Stelle des abgeschatteten Bereichs 204 in das zu korrigierende OCT-Bild 201 ein. Auf diese Weise erhält sie ein korrigiertes OCT-Bild 203, dessen Bereich 204 ersetzte Bildinformation 208 aufweist, die aus dem anderen OCT-Bild 202 stammt.

In der in Fig. 2 gezeigten Ausführungsform entfernt die Steuereinrichtung 29 zusätzlich im zu korrigierenden OCT-Bild 201 die Grenzfläche des Instrumentes 206. Dies ist optional.

Weiter optional ist die im korrigierten Bild 203 gezeigte Ausgestaltung, dergemäß die gesetzte Bildinformation 208 markiert wird, beispielsweise durch eine Graudarstellung. In der Schemazeichnung der Fig. 2 ist diese Markierung durch eine Punktierung im Bereich 204 symbolisiert. In einer Weiterbildung dieser Ausführungsform erfolgt die Markierung derart, dass sie einen Hinweis auf den zeitlichen Abstand zwischen dem zu korrigierenden OCT-Bild 201, das bei Online-Betrieb in der Regel ein aktuelles OCT-Bild ist, und dem anderen OCT-Bild 202, aus dem die Bildinformation 208 entnommen und im korrigierten OCT-Bild 203 ersetzt wurde. Auf diese Weise kann ein Benutzer, dem beispielsweise auf dem Bildschirm 83 das korrigierte OCT-Bild 203 angezeigt wird, einfach und intuitiv erkennen, dass die Abbildung des Objektes 7 im Bereich 204 ältere Bildinformationen enthält.
Die anhand der Fig. 2 gezeigte Bildkorrektur kann, wie bereits erwähnt, online, d.h. während laufender Erzeugung der Zeitreihe durch das OCT-Modul 115 ausgeführt werden. In einem solchen Fall wird es sich in der Regel beim zu korrigierenden OCT-Bild 201 um ein aktuelles OCT-Bild handeln. Die Bildverbesserung kann jedoch auch offline an bereits aufgenommenen Zeitreihen durchgeführt werden, die vom OCT-Bildmodul 115 zu einem früheren Zeitpunkt erzeugt und abgespeichert wurden.
Gemäß einem nicht beanspruchten Vergleichsbeispiel kann die Detektion des Instrumentes 206 und des daraus folgenden abgeschatteten Bereichs 204 ausschließlich anhand der OCT-Bilder, welche vom OCT-Bildmodul 115 bereitgestellt wurden, erfolgen. In einem derart vereinfachten Vergleichsbeispiel muss das Mikroskopiesystem keine optische Abbildung erzeugen. Mit anderen Worten, alle Elemente im Strahlengang oberhalb des Spiegels 69 können entfallen, auch das optische Bildmodul 101.
Die anhand des korrigierten Bildes 203 erläuterte Markierung der ersetzten Bildinformation 208 informiert einen Benutzer über Bildbereiche, deren Bildinformation möglicherweise eine geringere Zuverlässigkeit hat, da sie aus dem anderen Bild 202, also in der Regel aus einem älteren Bild stammt. Diese Information kann alternativ oder zusätzlich auch dadurch bereitgestellt werden, dass ein Korrekturinformationsbild 209 von der Steuereinrichtung 29 erzeugt wird. Das Korrekturinformationsbild 209 zeigt eine vereinfachte Darstellung des Objektes 7, in der der Bereich 204, in dem die ersetzte Bildinformation 208 verwendet wurde, und der aktuelle Bereich 210 unterschiedlich markiert werden. Die Markierung kann wiederum den zeitlichen Abstand zwischen dem anderen OCT-Bild 202 und dem zu korrigierenden OCT-Bild 201 kodieren, beispielsweise als Graustufen- oder Farbmarkierung.
Eine besonders bevorzugte Ausführungsform der Erfindung nutzt die Fähigkeit des Mikroskopiesystems 1, das zusätzlich zum OCT-Bild auch ein optisches Bild des Objektes 7 bereitstellt, erfindungsgemäß dahingehend, dass die Lage des abschattenden Instrumentes 206 und insbesondere die Lage des Schattens 205 und damit letztlich die Bestimmung des Bereiches 204 anhand des optischen Bildes erfolgt. Dies ist bereits deshalb vorteilhaft, da das optische Bild in der Regel eine sehr viel höhere Auflösung bereitstellt, als das OCT-Bild, was die Ermittlung des Bereiches 204 mit höherer Präzision erlaubt.

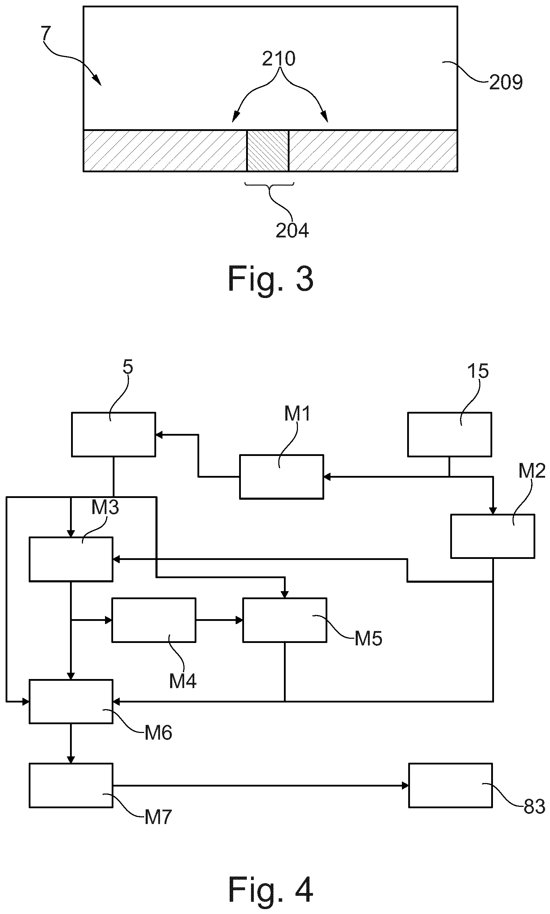

Fig. 4 bezieht sich auf diese bevorzugte, erfindungsgemäße Ausführungsform, wobei Elemente, die denen der Fig. 1 entsprechen, mit denselben Bezugszeichen versehen sind. Die Kamera 15 steht im Blockschaltbild der Fig. 4 exemplarisch für das optische Mikroskop, das mittels des optischen Bildmoduls 101 eine Abfolge von optischen Bildern erzeugt.

Die Abfolge von optischen Bildern erlaubt es zum einen in einem Stabilisierungsmodul M1 eine Ansteuerinformation für das OCT-System 5 zu gewinnen, die sicherstellt, dass das OCT-System 5 an einer gewünschten und bekannten Stelle, die auf das optische Bild bezogen ist, das OCT-Bild erzeugt. Dies ist auch deshalb vorteilhaft, da das OCT-Bild in der Regel eine sehr viel geringere Ausdehnung hat als das optische Bild.

Gleichzeitig wird in einem Instrumentenverfolgemodul M2 die Bewegung des Instrumentes im optischen Bild ermittelt. Das Instrumentenverfolgemodul M2 enthält dazu die optischen Bilddaten der Abfolge von optischen Bildern vom optischen Bildmodul 101. Das OCT-System 5 liefert die OCT-Bilddaten an ein Schattendetektionsmodul M3, das zusätzlich vom Instrumentenverfolgemodul M2 eine Information über die Lage des optischen Instruments bezogen auf das OCT-Bild erhält. Das Schattendetektionsmodul M3 ermittelt die Lage des Schattens 205 und damit letztlich des abgeschatteten Bereiches 204.

Die entsprechende Information wird in ein Speichermodul M4 eingeschrieben, das unter anderem die Zeitreihe enthält. Im Speichermodul M4 liegt damit eine Folge von Angaben über den abgeschatteten Bereich 204 in den OCT-Bildern vor. In einem Registrierungsmodul M5 werden die Angaben des Ortes des abgeschatteten Bereichs 204 bezüglich der aktuellen Position des OCT-Bilds zum OCT-System 5 registriert.

Ein Kompensationsmodul M6 erhält schließlich die aktuelle Angabe über den abgeschatteten Bereich 204 sowie die aus dem Speichermodul M5 mittels des Registrierungsmoduls M6 gewonnene Folge von Angaben über den abgeschatteten Bereich 204 in der Zeitreihe von OCT-Bildern. Zusammen mit der Information aus dem Instrumentenverfolgemodul M2 ermittelt das Kompensationsmodul M6 damit, wie bereits zuvor anhand der Fig. 2 erläutert, das jüngste andere Bild 201, in dem für den abgeschatteten Bereich 204 Bildinformation vom Objektiv vorliegt, und erzeugt das korrigierte OCT-Bild 203.

Ein Anzeigemodul M7 ermittelt den zeitlichen Abstand zwischen dem anderen OCT-Bild 202 und dem zu korrigierenden OCT-Bild 201 und markiert die ersetzte Bildinformation 208 entsprechend. Es gibt das entsprechende korrigierte Bild 203 (und soweit erzeugt auch das Korrekturinformationsbild 209) auf dem Bildschirm 83 aus.

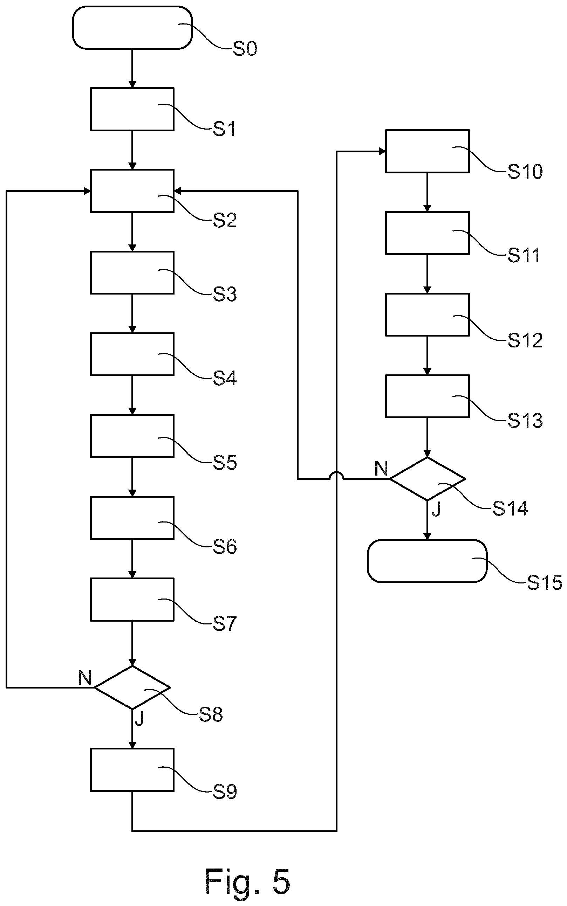

Fig. 5 zeigt ein Blockschaltbild eines Verfahrensablaufs zur Verbesserung eines OCT-Bildes hinsichtlich Abschattungen durch einen Gegenstand, der sich im Abbildungsstrahlengang der OCT-Bildgebung befindet. In einem Schritt S0 wird das Verfahren gestartet.

Ein Schritt S1 stellt eine Zeitreihe von OCT-Bildern bereit, beispielsweise durch Zugriff auf einen Speicher, der im Online-Betrieb OCT-Bilder zum Bilden einer Zeitreihe abspeichert, oder durch Einspielen einer entsprechenden Zeitreihe, die bei einem Offline-Betrieb früher aufgenommen wurde.

In einem Schritt S2 wird das zu korrigierende OCT-Bild 201 festgelegt. Bei einem Online-Betrieb wird es sich üblicherweise um das aktuellste OCT-Bild handeln. Die Auswahl des zu korrigierenden OCT-Bilds 201 kann aber auch durch einen Benutzer gesteuert werden, was insbesondere im Offline-Betrieb zweckmäßig ist.

In einem Schritt S3 wird das zum zu korrigierenden OCT-Bild zugehörige optische Bild erfasst oder ausgelesen. Nach einer Lageregistrierung im Schritt S4 zwischen optischem Bild und zu korrigierendem OCT-Bild 201 sucht ein Schritt S5 das abschattende Instrument im optischen Bild. Ein Schritt S6 ermittelt die Lage des Schattens im OCT-Bild 201 und dem von der Abschattung betroffenen Bildbereich 204. Ein Schritt S7 sucht dann ein älteres Bild ohne oder mit nicht vollständig abgeschattetem Bereich 204. Hierbei sei darauf hingewiesen, dass eine Bildverbesserung bereits dann erfolgt, wenn ein anderes Bild 202 gefunden wird, in dem der Bereich 204 zwar möglicherweise teilweise nicht jedoch vollständig abgeschattet ist. Dies ermöglicht bereits eine Verbesserung dahingehend, dass der Bereich 204, der von der Abschattung betroffen ist, verkleinert werden kann, nämlich auf einen Bereich, in dem der Bereich 207 des anderen OCT-Bilds 202 mit dem Bereich 204 des zu korrigierenden OCT-Bildes 201 überlappt.

Eine Sicherheitsabfrage im Schritt S8 überprüft, ob es gelang, ein solches anderes Bild zu finden. Nach einer direkten Aufnahme des Verfahrens im Online-Betrieb kann eine Situation auftreten, dass das Instrument 206 (noch) nicht bewegt wurde. In diesem Fall ist keine Bildverbesserung möglich und es wird (N-Verzweigung) zum Schritt S2 zurückgesprungen, mit der Maßgabe, dass dort ein anderes zu korrigierendes Bild S2 ausgewählt wird. Bei einem Online-Betrieb ist diese Anforderung durch die fortschreitende Aufnahme der Zeitreihe an OCT-Bildern unproblematisch erfüllbar. Bei einem Offline-Betrieb liegt ohnehin eine Zeitreihe von OCT-Bildern vor, so dass auch hier die Anforderung, ein anderes OCT-Bild für die Korrektur auszuwählen, sicher erfüllt werden kann.

Gelang es jedoch im Schritt S7 ein älteres OCT-Bild mit nicht vollständig abgeschattetem Bereich 204 zu finden (J-Verzweigung) wird in einem Schritt S9 die Bildinformation für diesen Bereich 204 aus dem anderen OCT-Bild 202 ausgelesen.

Ein Schritt S10 überschreibt für den Bereich 204 die Bildinformation im zu korrigierenden OCT-Bild 201, das im Schritt S1 festgelegt wurde, mit der ausgelesenen Bildinformation für den Bereich 204 aus dem anderen OCT-Bild 202, das im Schritt S7 aufgefunden wurde.

Dabei sei darauf hingewiesen, dass der Schritt S7 in einer bevorzugten Ausführungsform ein anderes OCT-Bild 202 sucht, das in möglichst geringem zeitlichen Abstand im zu korrigierenden OCT-Bild 201 aufgenommen wurde.

Durch die Einfügung der Bildinformation 208 in den Bereich 204 des zu korrigierenden OCT-Bilds 201 wird das korrigierte OCT-Bild 203 erzeugt. Ein Schritt S12 ermittelt den seitlichen Abstand zwischen dem anderen OCT-Bild 202 und dem zu korrigierenden OCT-Bild 201, d.h. den Altersabstand zwischen der in den Bereich 204 eingefügten Bildinformation 208 und der restlichen Abbildung des Objektes 7. Dieser Abstand wird dann in einem Schritt S13 zur Markierung der eingefügten Bildinformation 208 verwendet, wobei die Markierung den Altersabstand anzeigt, beispielsweise durch eine Graustufen- oder Farbcodierung.

In einem Schritt S14 wird abgefragt, ob das Verfahren beendet ist, d.h. ob weitere Bilder im Schritt S2 auszuwählen sind. Ist das Verfahren nicht beendet (N-Verzweigung), wird zum Schritt S2 zurückgesprungen, wiederum mit der Maßgabe, ein weiteres zu korrigierendes OCT-Bild 201 auszuwählen. Im Falle dass das Verfahren zu Ende ist (J-Verzweigung), wird das Verfahren im Schritt S15 beendet.

Folgende Modifikationen oder Abwandlungen bzw. spezielle Ausführungsformen sind möglich:

Wie bereits erwähnt, ist es vorteilhaft und erfindungsgemäß, die Detektion des Schattens 205 und die Ermittlung des im OCT-Bild von der Abschattung betroffenen Bereichs 204 in einem zugeordneten optischen Bild des Objektes 7 auszuführen. Aufgrund des größeren Bildfeldes kann dabei in einer optionalen Weiterbildung die Lage des abschattenden Gegenstandes verfolgt werden. Gemäß einem nicht beanspruchten Vergleichsbeispiel ist es jedoch gleichermaßen möglich, den Ort des Gegenstandes im OCT-Bild und damit den von der Abschattung betroffenen Bereich 204 direkt und ausschließlich im OCT-Bild zu ermitteln.

Das Verfahren kann online wie offline durchgeführt werden. In einem Online-Betrieb werden aktuelle OCT-Bilder verbessert, indem auf die Zeitreihe zugegriffen wird, welche frühere OCT-Bilder enthält. In einem Offline-Betrieb wird die Zeitreihe zur Verfügung gestellt, die beispielsweise früher aufgenommen wurde. Das Verfahren muss dann nicht in einem Mikroskopsystem 1 durchgeführt werden, sondern kann auch durch eine entsprechend programmierte Steuereinrichtung bewirkt werden. Auch im Offline-Betrieb ist es möglich, die Lage des von der Abschattung betroffenen Bereichs 204 entweder im OCT-Bild oder in einer zugehörigen Abfolge optischer Bilder zu ermitteln.

Die Verbesserung des OCT-Bildes ist vorstehend anhand eines Operationsmikroskops beschrieben, und der abschattende Gegenstand ist als chirurgisches Instrument erwähnt. Gleichermaßen ist jedoch eine Anwendung an totem Material gleichermaßen möglich, beispielsweise bei einer Autopsie oder der Untersuchung von technischem Material. Weiter wurde erläutert, dass das Instrument in das Objekt 7 eingeführt wird, während dies mit optischer Kohärenztomographie abgebildet wird. Die Bildverbesserung wird in einer Ausführungsform ebenso ausgeführt, wenn der Gegenstand sich außerhalb des Objektes 7 befindet.

In der Bauweise gemäß Fig. 1 ist das Mikroskopsystem als Kombinationsmikroskop ausgestaltet. Zwar erleichtert dies die Lageregistrierung von optischen Bildern und OCT-Bildern, jedoch ist die Verwendung optischer Bilder zur Verbesserung des OCT-Bildes gleichermaßen auch mit getrennten Mikroskopen, die sich weniger optische Elemente teilen, als es im Mikroskopsystem der Fig. 1 der Fall ist, oder gar keine.

In der Ausführungsform der Fig. 2 wird ein anderes OCT-Bild 202 gesucht, in dem der von der Abschattung betroffene Bereich 204 vollständig abgebildet ist. Dies ist, wie bereits erwähnt, der Optimalfall, da dann ein vollständig von Abschattung unbeeinträchtigtes korrigiertes Bild 203 erhalten wird. Wie bereits erwähnt, wird eine Bildverbesserung auch bereits dann erreicht, wenn ein anderes OCT-Bild 202 gefunden wird, in dem der Bereich 204 nur teilweise abgeschattet ist. In einer Weiterbildung ist es möglich, eine Gütefunktion zu definieren, die eine Abwägung zwischen dem Altersunterschied von anderem OCT-Bild 202 und zu korrigierendem OCT-Bild 201 und dem Grad der Abbildung des Bereiches 204 im anderen OCT-Bild 202 liefert. Auf diese Weise wird verhindert, dass für einen vollständigen Ersatz des Bereiches 204 durch Bildinformation 208 aus dem anderen OCT-Bild 202 auf ein sehr altes anderes OCT-Bild 202 zugegriffen wird. In einer Weiterbildung ist diese Gütefunktion vom Benutzer einstellbar, so dass er die Bedeutung einer möglichst vollständigen Abbildung des Objektes 7 oder einer möglichst aktuellen Abbildung des Objektes 7 gegeneinander abwägen kann.

In einer Ausführungsform wird verhindert, dass ein unerwünscht altes, anderes OCT-Bild 202 für die Gewinnung der Bildinformation 208 im abgeschatteten Bereich 204 herangezogen wird, indem ein Zeitfenster für die Zeitreihe definiert wird, innerhalb der das andere OCT-Bild 202 bezogen auf das zu korrigierende OCT-Bild 201 liegen muss. Die Definition eines solchen Zeitfensters ergänzt oder ersetzt die Verwendung der erwähnten Gütefunktion.

Das Verfahren beziehungsweise das Steuergerät des Kombinationsmikroskops umfasst/bewirkt in einem nicht beanspruchten Vergleichsbeispiel zwei Überprüfungsschritte. In einem ersten Schritt wird ein zu korrigierendes Bild daraufhin überprüft, ob es im OCT-Bild 201 einen Bereich 204 des Objektes 7 gibt, der durch den Gegenstand 206 abgeschattet wird. In einem zweiten Überprüfungsschritt wird ein anderes OCT-Bild 202 der Zeitreihe gesucht, in dem der im ersten Überprüfungsschritt ermittelte Bereich 204 nicht durch den Gegenstand 206 abgeschattet ist. Bei beiden Überprüfungsschritten kann eine Schwellwertüberprüfung verwendet werden. Zum Ermitteln des Bereiches 204, der durch den Gegenstand 206 abgeschattet wird, ist dann ein Referenzwert, der bei der Schwellwertüberprüfung zugrunde gelegt wird, genau so gewählt, dass damit eine Abschattung durch den Gegenstand, d. h. das Operationsinstrument, erkannt wird. Es ist deshalb für einige Ausführungsformen bevorzugt, dass bei der automatischen Suche der abgeschatteten Bereiche ein Schwellwert verwendet wird, der genau so gewählt wird/ist, dass damit ein Schatten, den der Gegenstand (z. B. ein Operationsinstrument) wirft, erkannt wird. Analog ist bei der Suche nach dem anderen OCT-Bild 202, in dem der Bereich 204 nicht durch den Gegenstand 206 abgeschattet ist, der Referenzwert so gewählt, dass damit eine mangelnde Abschattung durch den Gegenstand 206 erkannt wird.
Es kann sich bei beiden Überprüfungsschritten des nicht beanspruchten Vergleichsbeispiels um denselben Referenzwert handeln, beispielsweise einen Helligkeits- oder Intensitätsreferenzwert, der einmal unterschritten (1. Suche) und das andere Mal überschritten (2. Suche) werden muss. Ob eine Über- oder Unterschreitung vorliegt, ist letztlich von der Art des OCT-Bildes abhängig. Bei einem Negativbild lägen die Verhältnisse genau umgekehrt. Natürlich sind auch unterschiedliche Referenzwerte möglich, um eine Art Hysterese zu bewirken.

In beiden Überprüfungsschritten kann natürlich alternativ gemäß einer Ausführungsform der Erfindung die bereits erwähnte Detektion im optischen Bild des Objektes 7 verwendet werden, d. h. die abgeschatteten und/oder nicht-abgeschatteten Bereiche werden nicht im OCT-Bild ermittelt. Auch hierbei kann die genannte Schwellwertuntersuchung verwendet werden, wobei zusätzlich die Relativlage von optischem Bild und OCT-Bild und gegebenenfalls unterschiedliche Blickwinkel zu berücksichtigen sind. Letztlich wird dann aus dem optischen Bild die Lage des abgeschatteten Bereichs des OCT-Bilds ermittelt. Diese Lage kann als Zusatzinformation zum OCT-Bild abgespeichert werden, um eine spätere Offline-Analyse zu erreichen, oder das optische Bild wird zusätzlich zum OCT-Bild abgespeichert, sodass das gesamte Verfahren nachträglich, d. h. offline ausgeführt werden kann.

Selbstverständlich ist gemäß einer weiteren Ausführungsform der Erfindung auch eine Kombination der Schwellwertuntersuchung und der Analyse eines optischen Bildes möglich, beispielsweise kann für den ersten Überprüfungsschritt eine Untersuchung im OCT-Bild und für den zweiten Überprüfungsschritt die Verwendung eines optischen Bildes beziehungsweise der daraus abgeleiteten Information verwendet werden. Umgekehrtes ist gleichermaßen in Ausführungsformen möglich, d. h. die erste Überprüfung erfolgt anhand eines optischen Bildes und die zweite Überprüfung anhand des OCT-Bildes. Darüber hinaus ist es möglich, die Art der Überprüfung abhängig von weiteren Umständen, beispielsweise einer Angabe über den verwendeten Gegenstand zu wählen.

## Patentansprüche

1. Verfahren zum Verbessern eines OCT-Bildes (201) eines Objekts (7), wobei eine Zeitreihe von OCT-Bildern bereitgestellt wird, die vom Objekt (7) mittels optischer Kohärenztomographie durch einen Abbildungsstrahlengang (3) erzeugt wurden und, wobei das Verfahren zum Verbessern eines OCT-Bildes, um Abschattungen durch einen im Abbildungsstrahlengang bewegten Gegenstand (206), insbesondere ein Operationsinstrument, zu unterdrücken, verschiedene OCT-Bilder der Zeitreihe kombiniert,
wobei
- ein zu korrigierendes OCT-Bild (201) festgelegt wird,
- im zu korrigierenden OCT-Bild (201) ein Bereich (204) des Objektes (7) ermittelt wird, der durch den Gegenstand (206) abgeschattet wird,
- in der Zeitreihe ein anderes, bevorzugt älteres, OCT-Bild (202) gesucht wird, in dem der Bereich (204) des Objektes (7) nicht durch den Gegenstand (206) abgeschattet ist,
- aus diesem anderen OCT-Bild (202) für den Bereich (204) des Objektes (7) Bildinformation (208) ausgelesen wird und
- ein korrigiertes OCT-Bild (203) erzeugt wird, indem in das zu korrigierende OCT-Bild (201) die ausgelesene Bildinformation (208) eingefügt wird, wobei die Bildinformation (208) im zu korrigierenden OCT-Bild (201) den Bereich (204) des Objektes (7), der durch den Gegenstand (206) abgeschattet wird, ersetzt,
**dadurch gekennzeichnet, dass**
- das Objekt (7) zusätzlich optisch abgebildet und so ein optisches Bild des Objektes (7) erzeugt wird, wobei die Lage des Gegenstands (206) im optischen Bild festgestellt wird und daraus der Bereich (204) des Objektes (7) ermittelt wird, der durch den Gegenstand (206) im zu korrigierenden OCT-Bild (201) abgeschattet wird.

2. Verfahren nach Anspruch 1, **dadurch gekennzeichnet, dass** im korrigierten OCT-Bild (203) der Bereich (204) des Objektes (7), in den die Bildinformation (208) eingefügt wurde, markiert wird.

3. Verfahren nach Anspruch 2, **dadurch gekennzeichnet, dass** der Bereich (204) des Objektes (7), in den die Bildinformation (208) eingefügt wurde, hinsichtlich eines zeitlichen Abstands zwischen dem zu korrigierenden OCT-Bild (201) und dem anderen OCT-Bild (202) kodiert wird, insbesondere durch eine Graustufen- oder Farbdarstellung des Bereiches (204) des Objektes (7), in den die Bildinformation (208) eingefügt wurde, im korrigierten OCT-Bild (203).

4. Verfahren nach Anspruch 1, **dadurch gekennzeichnet, dass** ein Korrekturinformationsbild (209) erzeugt wird, das den Bereich (204) des Objektes (7), in den die Bildinformation (208) eingefügt wurde, anzeigt.

5. Verfahren nach einem der obigen Ansprüche , **dadurch gekennzeichnet, dass** eine Abfolge von optischen Bildern bereitgestellt wird, die parallel zur Zeitreihe von OCT-Bildern erzeugt wurde, wobei im Verfahren aus der Abfolge von optischen Bildern eine Folge von Angaben über den Bereich des Objektes, der durch den Gegenstand (206) im zu korrigierenden OCT-Bild (201) abgeschattet wird, ermittelt wird, wobei jeder Angabe der Folge das jeweilige zeitlich entsprechende OCT-Bild der Zeitreihe zugeordnet wird.

6. Verfahren nach einem der Ansprüche 1 bis 4, **dadurch gekennzeichnet, dass** der durch den Gegenstand (206) abgeschattete Bereich (204) mittels einer Schwellwertanalyse ermittelt wird, wobei ein Referenzwert, auf dessen Über- oder Unterschreitung geprüft wird, so gewählt ist, dass damit eine Abschattung durch den Gegenstand (206) erkannt wird.

7. Verfahren nach einem der Ansprüche 1 bis 4 oder 6, **dadurch gekennzeichnet, dass** bei der Suche des anderen Bildes (202), in dem der Bereich (204) des Objektes (7) nicht durch den Gegenstand (206) abgeschattet ist, eine Schwellwertüberprüfung verwendet wird, wobei ein Referenzwert verwendet wird, auf dessen Über- oder Unterschreitung geprüft wird, so gewählt ist, dass damit eine nicht erfolgte Abschattung durch den Gegenstand (206) erkannt wird.

8. Mikroskop zur Abbildung eines Objekts, wobei das Mikroskop (1) einen Abbildungsstrahlengang (3), der das Objekt (7) mittels optischer Kohärenztomographie (5) abbildet, und eine Bildbearbeitungseinrichtung (29) zur Unterdrückung von Abschattungen durch einen in einem Abbildungsstrahlengang (3) bewegten Gegenstand (206) in einer Zeitreihe von OCT-Bildern durch Kombination von OCT-Bildern der Zeitreihe aufweist,
wobei
- die Bildbearbeitungseinrichtung (29) ausgebildet ist, für ein zu korrigierendes OCT-Bild (201) einen in diesem OCT-Bild (201) liegenden Bereich (204) des Objektes (7) zu ermitteln, der durch den Gegenstand (206) abgeschattet ist,
- die Bildbearbeitungseinrichtung (29) ausgebildet ist, in der Zeitreihe ein anderes OCT-Bild (202) zu suchen, in dem der Bereich (204) des Objektes (7) nicht durch den Gegenstand (206) abgeschattet ist,
- die Bildbearbeitungseinrichtung (29) ausgebildet ist, aus diesem anderen OCT-Bild (202) für den Bereich (204) des Objektes (7) Bildinformation auszulesen und
- die Bildbearbeitungseinrichtung (29) ausgebildet ist, ein korrigiertes OCT-Bild (203) zu erzeugen, indem sie in das zu korrigierende OCT-Bild (201) die ausgelesene Bildinformation (208) einfügt, wobei die Bildinformation (208) im zu korrigierenden OCT-Bild (201) den Bereich (204) des Objektes (7), der durch den Gegenstand (206) abgeschattet ist, ersetzt,
**dadurch gekennzeichnet, dass**
- das Mikroskop (1) als Kombinationsmikroskop ausgebildet ist und eine Kamera (15) aufweist, die ein optisches Bild des Objektes (7) erzeugt und Optikbilddaten bereitstellt, wobei die Bildbearbeitungseinrichtung (29) ausgebildet ist, die Optikbilddaten auszulesen, die Lage des Gegenstands (206) im optischen Bild festzustellen und daraus den Bereich (204) des Objektes (7) zu ermitteln, der durch den Gegenstand (206) im zu korrigierenden OCT-Bild (201) abgeschattet ist.

9. Mikroskop nach Anspruch 8, **dadurch gekennzeichnet, dass**
die Bildbearbeitungseinrichtung (29) ausgebildet ist, im korrigierten OCT-Bild (203) den Bereich (204) des Objektes (7), in den die Bildinformation (208) eingefügt ist, zu markieren.

10. Mikroskop nach Anspruch 9, **dadurch gekennzeichnet, dass** die Bildbearbeitungseinrichtung (29) ausgebildet ist, den Bereich (204) des Objektes (7), in den die Bildinformation (208) eingefügt ist, hinsichtlich eines zeitlichen Abstand zwischen dem zu korrigierenden OCT-Bild (201) und dem anderen OCT-Bild (202) zu kodieren, insbesondere durch eine Graustufen- oder Farbdarstellung des Bereiches (204) des Objektes (7), in den die Bildinformation (208) eingefügt ist, im korrigierten OCT-Bild (203).

11. Mikroskop nach Anspruch 8, **dadurch gekennzeichnet, dass** die Bildbearbeitungseinrichtung (29) ausgebildet ist, ein Korrekturinformationsbild (209) zu erzeugen, das den Bereich (204) des Objektes (7), in den die Bildinformation (208) eingefügt ist, anzeigt.

12. Mikroskop nach einem der Ansprüche 8 bis 11, **dadurch gekennzeichnet, dass** die Bildbearbeitungseinrichtung (29) ausgebildet ist, den durch den Gegenstand (206) abgeschatteten Bereich (204) des Objektes (7) mittels einer Schwellwertanalyse zu ermitteln, wobei ein Referenzwert, auf dessen Über- oder Unterschreitung geprüft wird, so gewählt ist, dass damit eine Abschattung durch den Gegenstand (206) erkannt ist, und/oder dass die Bildbearbeitungseinrichtung (29) ausgebildet ist, bei der Suche des anderen OCT-Bildes (202), in dem der Bereich (204) des Objektes (7) nicht durch den Gegenstand (206) abgeschattet ist, eine Schwellwertanalyse zu verwenden, bei der ein Schwellwert, auf dessen Über- oder Unterschreitung geprüft wird, verwendet wird, der so gewählt ist, dass damit eine ausgebliebene Abschattung durch den Gegenstand (206) erkannt ist.

13. Mikroskop nach einem der Ansprüche 8 bis 12, **dadurch gekennzeichnet, dass** die Bildbearbeitungseinrichtung (29) ausgebildet ist, parallel zur Zeitreihe von OCT-Bildern eine Abfolge von optischen Bildern abzuspeichern und daraus eine Folge von Angaben über den Bereich (204) des Objektes (7), der durch den Gegenstand (206) abgeschattet ist, zu ermitteln, wobei jeder Angabe der Folge das jeweilige zeitlich entsprechende OCT-Bild der Zeitreihe zugeordnet ist.

## Claims

1. Method for improving an OCT image (201) of an object (7), wherein a time series of OCT images generated from the object (7) by means of optical coherence tomography by an imaging beam (3) is provided, and wherein the method for improving an OCT image, in order to suppress vignetting effects by an object (206) moved in the imaging beam, in particular a surgical instrument, combines various OCT images of the time series,
wherein
- an OCT image (201) to be corrected is established,
- a region (204) of the object (7) that is vignetted by the object (206) is determined in the OCT image (201) to be corrected,
- another, preferably older, OCT image (202), in which the region (204) of the object (7) is not vignetted by the object (206), is searched for in the time series,
- image information (208) for the region (204) of the object (7) is extracted from this other OCT image (202) and
- a corrected OCT image (203) is generated, in that the extracted image information (208) is inserted into the OCT image (201) to be corrected, wherein the image information (208) replaces the region (204) of the object (7) that is vignetted by the object (206) in the OCT image (201) to be corrected,
**characterized in that**
- the object (7) is additionally optically imaged and thus an optical image of the object (7) is generated, wherein the position of the object (206) in the optical image is established and is used to determine the region (204) of the object (7) that is vignetted by the object (206) in the OCT image (201) to be corrected.

2. Method according to Claim 1, **characterized in that** the region (204) of the object (7) into which the image information (208) has been inserted is marked in the corrected OCT image (203).

3. Method according to Claim 2, **characterized in that** the region (204) of the object (7) into which the image information (208) has been inserted is encoded with regard to a time interval between the OCT image (201) to be corrected and the other OCT image (202), in particular by a greyscale or colour representation of the region (204) of the object (7) into which the image information (208) has been inserted, in the corrected OCT image (203).

4. Method according to Claim 1, **characterized in that** a correction information image (209), which indicates the region (204) of the object (7) into which the image information (208) has been inserted, is generated.

5. Method according to the above claims, **characterized in that** a sequence of optical images that were generated in parallel with the time series of OCT images is provided, wherein the sequence of optical images is used in the method to determine a series of statements about the region of the object that is vignetted by the object (206) in the OCT image (201) to be corrected, wherein each statement of the series is assigned the respective temporally corresponding OCT image of the time series.

6. Method according to one of Claims 1 to 4, **characterized in that** the region (204) vignetted by the object (206) is determined by means of a threshold value analysis, wherein a reference value that is checked for being overshot or undershot is chosen such that vignetting by the object (206) is thereby detected.

7. Method according to one of Claims 1 to 4 or 6, **characterized in that** a threshold value check is used when searching for the other image (202), in which the region (204) of the object (7) is not vignetted by the object (206), wherein a reference value that is checked for being overshot or undershot is chosen such that absence of vignetting by the object (206) is thereby detected.

8. Microscope for imaging an object, wherein the microscope (1) has an imaging beam (3), which images the object (7) by means of optical coherence tomography (5), and an image processing device (29) for suppressing vignetting effects by an object (206) moved in an imaging beam (3) in a time series of OCT images by combining OCT images of the time series,
wherein
- the image processing device (29) is designed to determine for an OCT image (201) to be corrected a region (204) of the object (7) lying in this OCT image (201) that is vignetted by the object (206),
- the image processing device (29) is designed to search in the time series for another OCT image (202), in which the region (204) of the object (7) is not vignetted by the object (206),
- the image processing device (29) is designed to extract image information for the region (204) of the object (7) from this other OCT image (202) and
- the image processing device (29) is designed to generate a corrected OCT image (203), in that it inserts the extracted image information (208) into the OCT image (201) to be corrected, wherein the image information (208) replaces the region (204) of the object (7) that is vignetted by the object (206) in the OCT image (201) to be corrected,
**characterized in that**
- the microscope (1) is designed as a combined microscope and has a camera (15), which generates an optical image of the object (7) and provides optical image data, wherein the image processing device (29) is designed to extract the optical image data, to establish the position of the object (206) in the optical image and use it to determine the region (204) of the object (7) that is vignetted by the object (206) in the OCT image (201) to be corrected.

9. Microscope according to Claim 8, **characterized in that** the image processing device (29) is designed to mark in the corrected OCT image (203) the region (204) of the object (7) into which the image information (208) has been inserted.

10. Microscope according to Claim 9, **characterized in that** the image processing device (29) is designed to encode the region (204) of the object (7) into which the image information (208) has been inserted with regard to a time interval between the OCT image (201) to be corrected and the other OCT image (202), in particular by a greyscale or colour representation of the region (204) of the object (7) into which the image information (208) has been inserted, in the corrected OCT image (203).

11. Microscope according to Claim 8, **characterized in that** the image processing device (29) is designed to generate a correction information image (209), which indicates the region (204) of the object (7) into which the image information (208) has been inserted.

12. Microscope according to one of Claims 8 to 11, **characterized in that** the image processing device (29) is designed to determine by means of a threshold value analysis the region (204) of the object (7) vignetted by the object (206), wherein a reference value that is checked for being overshot or undershot is chosen such that vignetting by the object (206) is thereby detected, and/or **in that** the image processing device (29) is designed to use a threshold value analysis when searching for the other OCT image (202), in which the region (204) of the object (7) is not vignetted by the object (206), in which analysis the value used as a threshold value that is checked for being overshot or undershot is one chosen such that absence of vignetting by the object (206) is thereby detected.

13. Microscope according to one of Claims 8 to 12, **characterized in that** the image processing device (29) is designed to store in parallel with a time series of OCT images a sequence of optical images and to use this sequence to determine a series of statements about the region (204) of the object (7) that is vignetted by the object (206), wherein each statement of the series is assigned the respective temporally corresponding OCT image of the time series.

## Revendications

1. Procédé d'amélioration d'une image OCT (201) d'un objet (7), dans lequel une série temporelle d'images OCT ayant été générées par l'objet (7) au moyen d'une tomographie par cohérence optique est générée par l'intermédiaire d'un trajet de faisceau de formation d'image (3) et, dans lequel le procédé d'amélioration d'une image OCT combine des images OCT différentes de la série temporelle pour supprimer des masquages provoqués par un objet (206) déplacé dans le trajet de faisceau de formation d'image, en particulier un instrument opératoire, dans lequel
- une image OCT à corriger (201) est établie,
- une région (204) de l'objet (7) qui est masquée par l'objet (206) est déterminée dans l'image OCT à corriger (201),
- une autre image OCT (202), de préférence plus ancienne, dans laquelle la région (204) de l'objet (7) n'est pas masquée par l'objet (206) est recherchée dans la série temporelle, et
- des informations d'image (208) sont lues à partir de ladite autre image OCT (202) pour la région (204) de l'objet (7), et
- une image OCT corrigée (203) est générée en insérant les informations d'image (208) lues dans l'image OCT à corriger (201), dans lequel les informations d'image (208) contenues dans l'image OCT à corriger (201) remplacent la région (204) de l'objet (7) qui est masquée par l'objet (206),
- **caractérisé en ce qu'**une image de l'objet (7) est en outre formée optiquement et **en ce qu'**une image optique de l'objet (7) est ainsi générée, dans lequel la position de l'objet (206) dans l'image optique est établie et la région (204) de l'objet (7) qui est masquée par l'objet (206) dans l'image OCT à corriger (201) est déterminée à partir de ladite position.

2. Procédé selon la revendication 1, **caractérisé en ce que** la région (204) de l'objet (7) dans laquelle ont été insérées les informations d'image (208) est marquée dans l'image OCT corrigée (203).

3. Procédé selon la revendication 2, **caractérisé en ce que** la région (204) de l'objet (7) dans laquelle ont été insérées les informations d'image (208) est codée en ce qui concerne un intervalle de temps entre l'image OCT à corriger (201) et l'autre image OCT (202), en particulier au moyen d'une représentation en niveaux de gris ou en couleurs de la région (204) de l'objet (7) dans laquelle ont été insérées les informations d'image (208), dans l'image OCT corrigée (203).

4. Procédé selon la revendication 1, **caractérisé en ce qu'**une image d'informations de correction (209) qui indique la région (204) de l'objet (7) dans laquelle ont été insérées les informations d'image (208) est générée.

5. Procédé selon l'une des revendications précédentes, **caractérisé en ce qu'**une séquence d'images optiques ayant été générée parallèlement à la série temporelle d'images OCT est fournie, dans lequel une séquence de données concernant la région de l'objet qui est masquée par l'objet (206) dans l'image OCT à corriger (201) est déterminée par le procédé à partir de la séquence d'images optiques, dans lequel l'image OCT temporellement correspondante respective de la série temporelle est associée à chaque donnée de la séquence.

6. Procédé selon l'une des revendications 1 à 4, **caractérisé en ce que** la région (204) masquée par l'objet (206) est déterminée par une analyse de valeur de seuil, dans lequel une valeur de référence est sélectionnée de telle sorte qu'un masquage provoqué par l'objet (206) est détecté lorsqu'il est vérifié que celle-ci est franchie en sens croissant ou décroissant.

7. Procédé selon l'une des revendications 1 à 4 ou 6, **caractérisé en ce que**, lors de la recherche de l'autre image (202) dans laquelle la région (204) de l'objet (7) n'est pas masquée par l'objet (206), un test de valeur de seuil est utilisé, dans lequel on utilise une valeur de référence qui est sélectionnée de telle sorte qu'une absence de masquage provoqué par l'objet (206) est détectée lorsqu'il est vérifié que celle-ci est franchie en sens croissant ou décroissant.

8. Microscope destiné à former une image d'un objet, dans lequel le microscope (1) comporte un trajet de faisceau de formation d'image (3) qui forme une image de l'objet (7) par tomographie à cohérence optique (5), et un dispositif de traitement d'image (29) destiné à supprimer des masquages provoqués par un objet (206) déplacé dans un trajet de faisceau de formation d'image (3) dans une série temporelle d'images OCT en combinant des images OCT de la série temporelle, dans lequel
- le dispositif de traitement d'image (29) est conçu pour déterminer, pour une image OCT à corriger (201), une région (204) de l'objet (7) située dans ladite image OCT (201), qui est masquée par l'objet (206),
- le dispositif de traitement d'image (29) est conçu pour rechercher dans la série temporelle une autre image OCT (202) dans laquelle la région (204) de l'objet (7) n'est pas masquée par l'objet (206),
- le dispositif de traitement d'image (29) est conçu pour lire des informations d'image à partir de ladite autre image OCT (202) pour la région (204) de l'objet (7), et
- le dispositif de traitement d'image (29) est conçu pour générer une image OCT corrigée (203) en insérant les informations d'image lues (208) dans l'image OCT à corriger (201), dans lequel les informations d'image (208) remplacent, dans l'image OCT à corriger (201), la région (204) de l'objet (7) qui est masquée par l'objet (206), **caractérisé en ce que**
- le microscope (1) est réalisé sous la forme d'un microscope combiné qui comporte une caméra (15) générant une image optique de l'objet (7) et fournissant des données d'image optiques, dans lequel le dispositif de traitement d'image (29) est conçu pour lire les données d'image optiques, pour établir la position de l'objet (206) dans l'image optique et pour déterminer à partir de celle-ci la région (204) de l'objet (7) qui est masquée par l'objet (206) dans l'image OCT à corriger (201).

9. Microscope selon la revendication 8, **caractérisé en ce que** le dispositif de traitement d'image (29) est conçu pour marquer, dans l'image OCT corrigée (203), la région (204) de l'objet (7) dans laquelle sont insérées les informations d'image (208).

10. Microscope selon la revendication 9, **caractérisé en ce que** le dispositif de traitement d'image (29) est conçu pour coder la région (204) de l'objet (7) dans laquelle sont insérées les informations d'image (208) en ce qui concerne un intervalle de temps entre l'image OCT à corriger (201) et l'autre image OCT (202), en particulier par une représentation en niveaux de gris ou en couleurs de la région (204) de l'objet (7) dans laquelle sont insérées les informations d'image (208), dans l'image OCT corrigée (203).

11. Microscope selon la revendication 8, **caractérisé en ce que** le dispositif de traitement d'image (29) est conçu pour générer une image d'informations de correction (209) indiquant la région (204) de l'objet (7) dans laquelle sont insérées les informations d'image (208).

12. Microscope selon l'une des revendications 8 à 11, **caractérisé en ce que** le dispositif de traitement d'image (29) est conçu pour déterminer la région (204) de l'objet (7) masquée par l'objet (206) au moyen d'une analyse de valeur de seuil, dans lequel une valeur de référence est sélectionnée de telle sorte qu'un masquage produit par l'objet (206) est détecté lorsqu'il est vérifié que celle-ci est franchie en sens croissant ou décroissant et/ou **en ce que** le dispositif de traitement d'image (29) est conçu, lors de la recherche de l'autre image OCT (202) dans laquelle la région (204) de l'objet (7) n'est pas masquée par l'objet (206), pour utiliser une analyse de valeur de seuil lors de laquelle on utilise une valeur de seuil qui est sélectionnée de telle sorte qu'une absence de masquage par l'objet (206) est détectée lorsqu'il est vérifié que celle-ci est franchie en sens croissant ou décroissant.

13. Microscope selon l'une des revendications 8 à 12, **caractérisé en ce que** le dispositif de traitement d'image (29) est conçu pour stocker une séquence d'images optiques en parallèle avec la série temporelle d'images OCT et pour déterminer à partir de celle-ci une séquence de données concernant la région (204) de l'objet (7) qui est masquée par l'objet (206), dans lequel l'image OCT temporellement correspondante respective de la série temporelle est associée à chaque donnée de la séquence.
